# FASCICULE DE BREVET EUROPEEN

(11) **EP 1 928 355 B1**
(45) Date de publication et mention de la délivrance du brevet: **16.11.2011**
(21) Numéro de dépôt: 06808220.5
(22) Date de dépôt: 29.09.2006
(51) Int. Cl.: A61F 2/24

(54) **PROTHESE POUR SEGMENT AORTIQUE ASCENDANT**
PROTHESE FÜR EIN AUFSTEIGENDES AORTENSEGMENT
PROSTHESIS FOR ASCENDING AORTIC SEGMENT

(30) Priorité: 29.09.2005 FR 0509954
(43) Date de publication de la demande: 11.06.2008
(73) Titulaire: Fabiani, Jean-Noel, 75015 Paris (FR)
(72) Inventeur: Fabiani, Jean-Noel, 75015 Paris (FR)
(74) Mandataire: Remy, Vincent Noel Paul
(86) Numéro de dépôt international: PCT/FR2006/002210
(87) Numéro de publication internationale: WO 2007/036644

(56) Documents cités:
- WO-A-95/03754
- WO-A-2005/000168
- FR-A- 2 688 692
- US-A1- 2005 065 597
- US-B1- 6 375 679
- US-B1- 6 544 285

## Description

La présente invention concerne une prothèse pour segment aortique ascendant et un procédé de traitement chirurgical d'une fuite valvulaire aortique.

L'aorte est la plus grosse artère du corps humain, qui prend son origine au niveau du coeur et chemine tout au long de la face antérieure de la colonne vertébrale. A partir de celle-ci, de nombreuses artères permettent d'alimenter l'ensemble du corps humain.

L'orifice de l'aorte, du côté coeur, est fermé par une valvule constituée de trois valves aortiques (valve postérieure, antérieure gauche et antérieure droite) qui sont des replis membraneux ou sigmoïdes (en nid de pigeon) en forme de demi-corbeille avec une face convexe. Elles possèdent chacune à leurs extrémités supérieures, un nodule d'Arantius et la réunion des trois nodules d'Arantius permet une obturation totale de l'orifice.

Les valves aortiques s'écartent les unes des autres en systole et viennent s'adosser en diastole (lorsque le courant est inversé) pour occlure l'orifice correspondant de manière à éviter un reflux.

Elles assurent ainsi l'étanchéité des ventricules lors de la diastole (relâchement) pour éviter le reflux du sang de l'aorte ascendante au ventricule gauche.

Cependant, il arrive que la valvule se dilate et que les valves aortiques, écartées les unes des autres, ne parviennent plus à fermer correctement l'orifice.

Or, une fermeture incomplète des valves aortiques aboutit à un reflux de sang de l'aorte vers le ventricule gauche, appelé fuite valvulaire, au moment de la diastole, entraînant une dilatation du ventricule, ce qui a pour conséquence immédiate une insuffisance hémodynamique du fait de la surcharge de sang dans le ventricule.

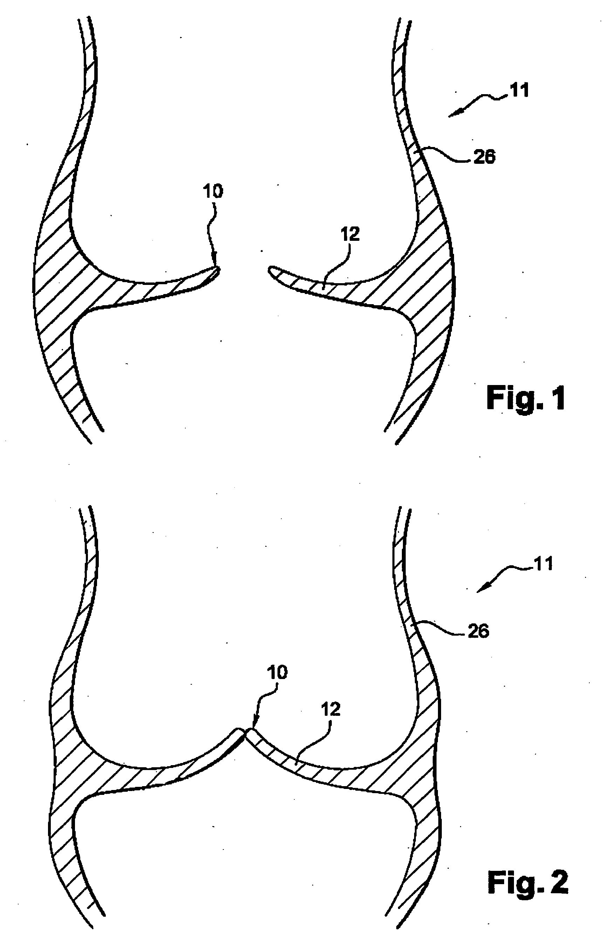

Ce cas de figure est illustré aux figures 1 et 2, sur lesquelles on voit des nodules d'Arantius 10 qui devraient permettre l'obturation totale de l'aorte 11. Ces nodules contrôlent des valves aortiques 12 qui ont normalement une forme de demi-corbeille.

Dans la configuration présentée sur la figure 1, les valves aortiques 12 ont perdu de leur convexité et sont ainsi amenées à ne plus se fermer correctement.

Dans la configuration présentée à la figure 2, les valves aortiques 12 se ferment correctement.

Pour traiter une telle insuffisance hémodynamique, le chirurgien peut soit décider de changer radicalement la valvule aortique, soit décider de réparer la valvule malade.

Cependant, dans la mesure du possible, le chirurgien préfère conserver les valves aortiques (c'est la chirurgie réparatrice ou plastie valvulaire) quand l'état de celles-ci le permet.

On connaît déjà, dans l'état de la technique illustré par le document FR-A-2688692, une technique de réparation des valves aortiques naturelles.

Pour cela, on utilise une armature filiforme reproduisant la forme trifoliée de l'orifice valvulaire aortique. Cette armature filiforme est munie d'un cordon suturable permettant de la solidariser à l'orifice valvulaire, à la base des valves aortiques. Cette opération permet de resserrer les valves aortiques 12, lesquelles reprennent ainsi leur forme de demi-corbeille.

Cependant, à la suite d'une fuite valvulaire, il est fréquent que le segment ascendant de l'aorte ne puisse plus assurer le maintien de la pression artérielle et laisse apparaître un anévrisme qui est une dilatation d'un segment de l'aorte issue d'altérations structurales de la paroi aortique.

Dans ce cas, il faut réopérer le patient pour lui poser une prothèse pour segment aortique ascendant.

Actuellement, pour traiter ces pathologies, on assemble une prothèse pour segment aortique ascendant avec une prothèse valvulaire mécanique ou avec une bioprothèse. Toutefois, l'implantation d'une prothèse mécanique, comme dans WO 95/03754, nécessite un traitement post-opératoire à l'aide d'anticoagulants, traitement qui peut s'avérer contraignant pour certaines catégories de patients (enfants ou adolescents, jeune femme souhaitant avoir des enfants...).

En outre, l'implantation directe d'une combinaison d'une bioprothèse et d'une prothèse pour segment aortique ascendant n'est pas possible en raison des conditions incompatibles de conservation de ces deux types de prothèse (fibres synthétiques en Dacron et matière biologique) à l'extérieur du corps humain. Il est donc difficile de faire coexister une telle combinaison. Pour palier ce dernier inconvénient, il est d'ailleurs actuellement recherché une alternative à la prothèse pour segment aortique en Dacron.

L'invention a notamment pour but de remédier à cet inconvénient en fournissant une prothèse pour segment aortique ascendant permettant d'effectuer une plastie valvulaire aortique et de prévenir un anévrisme de l'aorte.

A cet effet, l'invention a pour objet une prothèse pour segment aortique ascendant caractérisée en ce qu'elle est constituée par un tube ayant une extrémité découpée selon la forme trifoliée d'un orifice valvulaire et en ce que ledit tube est solidaire, à cette extrémité, d'une armature filiforme s'étendant suivant un contour fermé conformé sensiblement comme un anneau natif de valve aortique saine, l'armature filiforme étant liée à une zone de suture s'étendant le long de l'armature filiforme tout au long du contour fermé.

L'invention répond d'une façon nouvelle au besoin de traiter une fuite valvulaire tout en prévenant un anévrisme de l'aorte. En effet, la prothèse selon l'invention étant susceptible d'être réalisée entièrement en matériau synthétique, par exemple en Dacron, sa conservation à l'extérieur du corps humain est possible. Une conséquence importante pour le patient est que l'intervention chirurgicale peut être de durée réduite.

En outre, la pose d'une prothèse selon l'invention est peu délabrante pour le corps humain car elle ne nécessite pas la mise oeuvre d'étapes traumatisantes comme la dissection des artères coronaires.

De plus, la prothèse selon l'invention permet une conservation des valves natives, ce qui évite les changements fréquents de prothèse le long de la vie du patient et, surtout, permet d'éviter de nombreux traitements post-opératoires, tel que la prise d'anticoagulants, à la suite de l'implantation de valves artificielles. Les risques de complications dues à une mauvaise prise du traitement post-opératoire sont donc réduits. Par ailleurs, en cas d'opérations sur des enfants en bas âge, pour lesquels la prothèse selon l'invention doit être replacée après quelques années en raison de la croissance de l'aorte, le remplacement peut intervenir plus tard que si une prothèse valvulaire avait été implantée car l'armature filiforme n'obstrue pas l'aorte à l'ouverture des valves, contrairement aux prothèses artificielles.

Avantageusement, la prothèse pour segment aortique ascendant est similaire à une prothèse de Valsalva, laquelle présente l'avantage d'avoir une embase élargie de forme similaire aux sinus de Valsalva.

Suivant une autre caractéristique optionnelle, la solidarisation de l'armature filiforme à l'extrémité du tube prothétique est réalisée par suture ou soudure thermique.

L'invention a également pour objet un procédé de traitement chirurgical d'une fuite valvulaire aortique due à un anneau aortique malade, **caractérisé en ce qu'**il consiste à implanter une prothèse, telle que décrite ci-dessus, comprenant notamment une armature filiforme s'étendant suivant un contour fermé conformé sensiblement comme un anneau natif de valve aortique, et à suturer sur l'anneau aortique natif malade, la zone de suture liée à l'armature de la prothèse.

Suivant d'autres caractéristiques optionnelles de la prothèse selon l'invention :
- l'armature filiforme est gainée de fibres en polyester ;
- les fibres en polyester sont du type de celles commercialisées sous la marque Dacron, propriété de Dupont de Nemours;
- l'armature filiforme est souple de manière à faciliter l'insertion de celle-ci dans l'aorte ;
- l'armature filiforme est en alliage inoxydable, tel que celui commercialisé sous la marque Elgiloy par la société Elgiloy Corporation ;
- la zone de suture est un cordon en Dacron ;
- la solidarisation de l'armature filiforme et de la zone de suture est assurée par une enveloppe en Dacron.

L'invention sera mieux comprise à la lecture de la description qui va suivre, donnée uniquement à titre d'exemple et faite en se référant aux dessins dans lesquels :
- la figure 1 est une vue en coupe d'une valvule aortique malade ;
- la figure 2 est une vue en coupe d'une valvule aortique saine ;
- la figure 3 représente, en perspective, une armature filiforme gainée d'une prothèse annulaire pour plastie valvulaire aortique selon un mode de réalisation de l'invention ;
- la figure 4 est une coupe transversale selon IV-IV de l'armature filiforme gainée de la figure 3 ;
- la figure 5 représente une prothèse selon un mode de réalisation de l'invention comprenant l'armature filiforme de la figure 3 ;
- la figure 6 représente la prothèse de la figure 5 en place sur un anneau aortique natif malade.

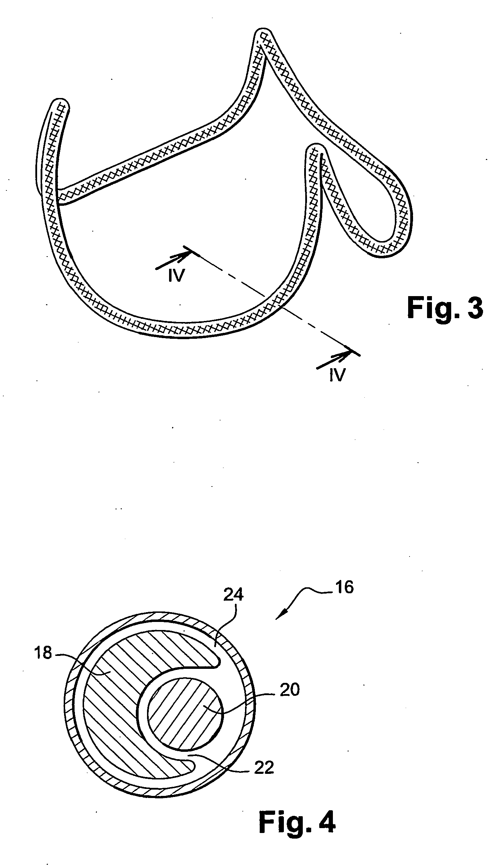

On va décrire ci-dessous, en se référant aux figures 3 à 6, une armature filiforme 18. Sur les figures 3 à 6, les éléments analogues à ceux des figures 1 et 2 sont désignés par des références identiques.

Une armature filiforme 18 s'étend selon un contour fermé conformé sensiblement comme un anneau natif d'une valve aortique saine.

L'armature filiforme 18 est en alliage inoxydable, tel que celui commercialisé sous la marque Elgiloy par la société Elgiloy Corporation.

Comme représenté sur la figure 4, une zone de suture 20 matérialisée par un cordon en Dacron s'étend le long de l'armature filiforme 18, laquelle comporte à cet effet un dégagement longitudinal 22 ménagé dans sa section circulaire. Cette zone de suture 20 permet le passage des points de chirurgie pour suturer l'armature gainée 16 à des valves aortiques natives malades.

La zone de suture 20 et l'armature filiforme 18 sont rendues solidaires l'une de l'autre, tout au long du contour fermé, par une enveloppe 24 en Dacron.

L'armature filiforme 18 souple ainsi gainée de fibres en polyester forme une armature gainée 16.

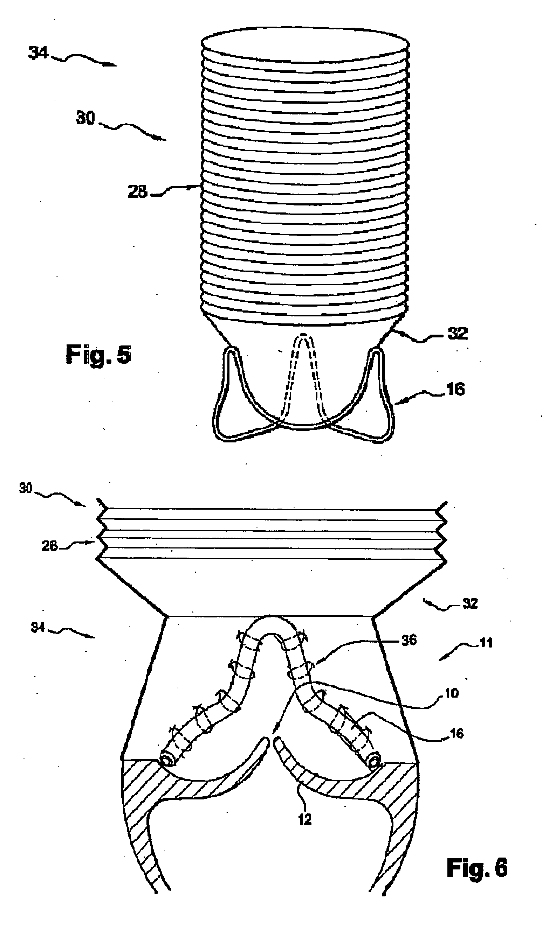

Comme on le voit sur la figure 5, l'armature gainée 16 est reliée à un tube 28 pour former une prothèse pour segment aortique ascendant 34.

Le tube 28 comporte une partie haute 30 sensiblement tubulaire en Dacron et une partie basse 32 en forme de cône, adaptée au diamètre de la partie haute 30 et au diamètre de l'armature gainée 16.

Une extrémité inférieure (par rapport au dessin) de la partie basse 32 est conformée selon le même contour fermé que l'armature gainée 16 et est solidarisée à ladite armature gainée 16 par suture ou soudure thermique.

La prothèse 34, comprenant l'armature filiforme gainée 16 et le tube 28, assure à la fois une réduction homogène de la valve aortique dilatée et le remplacement du segment aortique malade.

On a représenté, sur la figure 6, un mode de mise en oeuvre du procédé de traitement chirurgical d'une fuite valvulaire aortique à l'aide de la prothèse 34.

Après avoir retiré le segment aortique ascendant, on a implanté la prothèse 34 en appliquant l'armature gainée 16 contre l'anneau aortique natif malade de l'aorte 11. On a ensuite suturé l'armature gainée 16 audit anneau tout le long du contour fermé grâce à des noeuds 36.

Grâce à l'armature, les valves aortiques 12 retrouvent leur forme convexe. En outre, le tube 28 remplace le segment aortique ascendant retiré, ce qui prévient tout risque d'anévrisme de l'aorte.

L'armature gainée 16 est ici solidarisée à la valvule par l'intérieur, ce qui offre l'avantage que l'aorte 11 n'a pas besoin d'être sectionnée.

## Revendications

1. Prothèse (34) pour segment aortique ascendant constituée par un tube (28) **caractérisée en ce que** ledit tube a une extrémité découpée selon la forme trifoliée d'un orifice valvulaire et **en ce que** ledit tube est solidaire, à cette extrémité, d'une armature filiforme (18) s'étendant suivant un contour fermé conformé sensiblement comme un anneau natif de valve aortique saine, l'armature filiforme (18) étant liée à une zone de suture s'étendant le long de l'armature filiforme (18) tout au long du contour fermé.

2. Prothèse (34) selon la revendication 1, dans laquelle l'armature filiforme (18) est gainée de fibres en polyester.

3. Prothèse (34) selon l'une quelconque des revendications 1 et 2, dans laquelle les fibres en polyester sont du type polyéthylène térephtalate.

4. Prothèse (34) selon l'une quelconque des revendications précédentes, dans laquelle l'armature filiforme (18) est en alliage inoxydable, tel qu'un alliage à base de cobalt, nickel et chrome.

5. Prothèse (34) selon l'une quelconque des revendications précédentes, dans laquelle la zone de suture (20) est un cordon entextile de fibres de polyéthylène térephtalate.

6. Prothèse (34) selon l'une quelconque des revendications précédentes, dans laquelle la solidarisation de l'armature filiforme (18) et de la zone de suture (20) est assurée par une enveloppe (24) en textile de fibres de polyéthylène térephtalate.

7. Prothèse (34) selon l'une quelconque des revendications précédentes, dans laquelle le tube est similaire à une prothèse de Valsalva.

8. Prothèse (34) selon l'une quelconque des revendications précédentes, dans laquelle l'armature filiforme (18) est solidarisée au tube (28) par suture ou soudure thermique.

## Claims

1. Prosthesis (34) for an ascending aortic segment, consisting of a tube (28) **characterized in that** said tube has one end cut out to match the trifoliate shape of a valve opening, and that said tube is secured, at this end, to a wire frame (18) extending along a closed outline substantially shaped like the ring of a healthy native aortic valve, the wire frame (18) being linked to a suture zone extending along the entire length of the closed outline of the wire frame (18).

2. Prosthesis (34) in accordance with claim 1 in which the wire frame (18) is sheathed with polyester fibers.

3. Prosthesis (34) in accordance with claims 1 and 2 in which the polyester fibers are of the type polyethylene terephtalate.

4. Prosthesis (34) in accordance with any of the preceding claims, in which the wire frame (18) is made of a stainless steel alloy such as an alloy with cobalt, nickel and chromium.

5. Prosthesis (34) in accordance with any of the preceding claims, in which the suture zone consists of a string in fabric made of polyethylene terephtalate fibers.

6. Prosthesis (34) in accordance with any of the preceding claims, in which the wire frame is firmly attached to the suture zone by an envelope in fabric made of polyethylene terephtalate fibers.

7. Prosthesis (34) in accordance with any of the preceding claims, in which the tube is similar to a Valsalva prosthesis.

8. Prosthesis (34) in accordance with any of the preceding claims, in which the wire frame (18) is firmly attached to the tube (28) by sutures or welding.

## Patentansprüche

1. Prothese (34) für ein aufsteigendes Aortensegment, die von einer Röhre (28) gebildet wird, **dadurch gekennzeichnet, dass** die Röhre ein Ende aufweist, das gemäß der Trifoliumform einer Klappenöffnung zugeschnitten ist, und dass die Röhre, an diesem Ende, mit einem drahtförmigen Gestell (18) fest verbunden ist, das sich einer geschlossenen Kontur folgend erstreckt, die im Wesentlichen wie ein nativer Anulus einer gesunden Aortenklappe geformt ist, wobei das drahtförmige Gestell (18) mit einem Nahtbereich verbunden ist, der sich entlang dem drahtförmigen Gestell (18) über die gesamte Länge der geschlossenen Kontur erstreckt.

2. Prothese (34) nach Anspruch 1, wobei das drahtförmige Gestell (18) mit Fasern aus Polyester ummantelt ist.

3. Prothese (34) nach einem der Ansprüche 1 und 2, wobei die Fasern aus Polyester vom Typ Polyethylenterephthalat sind.

4. Prothese (34) nach einem der vorstehenden Ansprüche, wobei das drahtförmige Gestell (18) aus einer nicht rostenden Legierung, wie einer Legierung auf Kobalt-, Nickel- und Chrombasis, ist.

5. Prothese (34) nach einem der vorstehenden Ansprüche, wobei der Nahtbereich (20) eine textile Schnur aus Polyethylenterephthalatfasern ist.

6. Prothese (34) nach einem der vorstehenden Ansprüche, wobei die feste Verbindung des drahtförmigen Gestells (18) und des Nahtbereichs (20) durch eine textile Ummantelung (24) aus Polyethylenterephthalatfasern gewährleistet ist.

7. Prothese (34) nach einem der vorstehenden Ansprüche, wobei die Röhre einer Valsalva-Prothese ähnelt.

8. Prothese (34) nach einem der vorstehenden Ansprüche, wobei das drahtförmige Gestell (18) mit der Röhre (28) durch Naht oder Thermoschweißung fest verbunden ist.
